(19) Europäisches Patentamt
European Patent Office
Office européen des brevets

(11) EP 2 935 208 B1

(12) **EUROPEAN PATENT SPECIFICATION**

(45) Date of publication and mention of the grant of the patent:
**24.07.2019 Bulletin 2019/30**

(21) Application number: **13814636.0**

(22) Date of filing: **22.11.2013**

(51) Int Cl.:
***C07C 305/04*** (2006.01) ***C07C 305/10*** (2006.01)
***C07C 303/24*** (2006.01) ***A61P 31/04*** (2006.01)
***A61P 39/04*** (2006.01)

(86) International application number:
**PCT/ID2013/000009**

(87) International publication number:
**WO 2014/097284 (26.06.2014 Gazette 2014/26)**

(54) **SULPHATED CHELATING AGENTS**

SULFATIERTE CHELATBILDNER

AGENTS CHÉLATANTS SULFATÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2012 ID 20121174**

(43) Date of publication of application:
**28.10.2015 Bulletin 2015/44**

(73) Proprietors:
- **Wardoyo, Haryanto**
  **Tangerang (ID)**
- **Hadipraja, Bobby**
  **Jakarta 14240 (ID)**

(72) Inventor: **WARDOYO, Haryanto**
**Kecamatan Serpong,**
**Tangerang (ID)**

(74) Representative: **V.O.**
**P.O. Box 87930**
**2508 DH Den Haag (NL)**

(56) References cited:
**WO-A1-02/081487 US-A1- 2008 227 987**

- **PETER CLAESSON: "Ueber die Aetherschwefelsäuren der mehrsäurigen Alkohole und der Kohlehydrate nebst einigen Bemerkungen über die Constitution der letzteren", JOURNAL FUER PRAKTISCHE CHEMIE, vol. 19, no. 2, 1879, pages 1-34, XP002720747, LEIPZIG ISSN: 0021-8383**
- **KREMS I J ET AL: "The Reaction of Lauric Acid Esters with Sulfuric Acid", JOURNAL OF THE AMERICAN CHEMICAL SOCIETY, ACS PUBLICATIONS, US, vol. 81, 1 January 1959 (1959-01-01), pages 1620-1627, XP002222376, ISSN: 0002-7863, DOI: 10.1021/JA01516A028**
- **M. SCHMIDT ET AL: "Zur Struktur von Hydroxy-5-phenyl-furanonen: Lösungs-NMR, Festkörper-13C-NMR und Röntgenstruktur im Vergleich / On the Structure of Hydroxy-5-phenyl-furanone Derivatives: NMR Spectroscopy in Solution and High Resolution Solid State 13C NMR Spectroscopy in Relation to X-Ray Crystallography", ZEITSCHRIFT FUR NATURFORSCHUNG- SECTION B JOURNAL OF CHEMICAL SCIENCES, vol. 48, no. 10, 1 January 1993 (1993-01-01), pages 1372-1380, XP055282423, ISSN: 0932-0776, DOI: 10.1515/znb-1993-1011**
- **D. G. DAVIES: "The Involvement of Cell-to-Cell Signals in the Development of a Bacterial Biofilm", SCIENCE, vol. 280, no. 5361, 10 April 1998 (1998-04-10), pages 295-298, XP055403018, ISSN: 0036-8075, DOI: 10.1126/science.280.5361.295**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 935 208 B1

## Description

### Field of the invention

This invention relates to sulphate chelating agents which have the ability to enlarge the membrane porosity of simple microorganism cell membranes and other organic membranes and to methods of synthesis of the agents.

### Background of the invention

Many types of chelating agents are commonly known and have been applied in various industries. Chelating agents are chemicals which contain donor atoms that have the ability to bind metallic atoms in a coordinate complex bond that turn into cyclic structural substance known as chelating complex or in its simple term: a chelate. Chelation technology was developed from naturally occurring chemicals that contain naturally or purposely added metallic ions. The use of chelating agent might give a way to control or manipulate the metallic ions in the system to perform the expected effect. The chelating complex substance formed from the interaction of some metallic ion with some chelating agent use to have significantly different characteristics/properties either from its original ions or the chelating agent itself. Therefore its characteristics or properties could be modified.

Therefore, the chelating agents are very effective substances in the formation of the complex substance with the metallic cations and also with the organic salts in order to prevent them act as simple hydrated cations. Common example of chelating agent is ethylenediaminetetraacetic acid (EDTA) (1) and its derivatives that will form the complex substance with most of $M^{2+}$ and $M^{3+}$ types of cations.

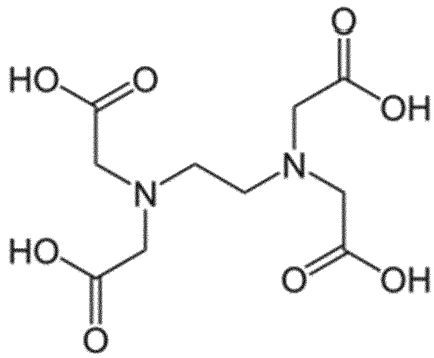

**EDTA**

It has been known also that gluconic acid and some others hydroxide acid perform similar properties.

There are many features of the chelating reaction that become the basic of chelating agents various applications.

The first, chelation provides a mechanism to control the concentration of the free metallic ions via dissociation equilibrium between chelating agent and said metallic ions. A related application is the sequestration process that reduces several properties of some metal without eliminating it from the existing system or phase; solubilization is a process in which an elaborate undissolved component phase becomes soluble in the said medium, and daparing, a condition where the addition or removal of several metallic ions result in insignificant change of the said ionic concentration inside the solution which totally depends on the accurate control of the chelating agent concentration.

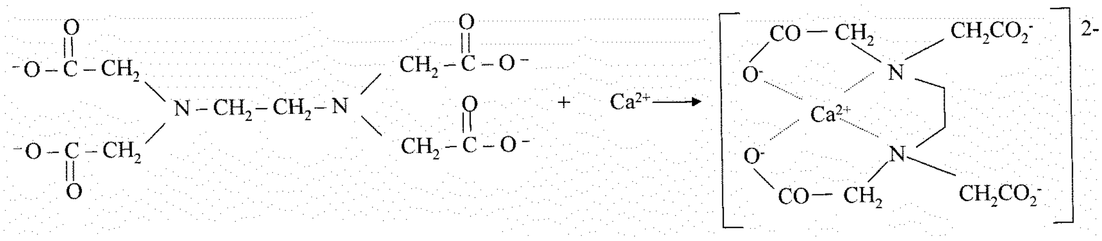

All of the above features and properties have been chemically applied in industry.

For example, the sequestration of the metallic ions could be used to control the water hardness. Then solubilization is used to dissolve the boiler scale, heat exchange equipment scale and the hard scale in the pipe.

In the mining industry, solubilization utilizes chelating agents to extract the metal from the metal ores, also to

be used to clean the contaminated area.

Daparing with the chelating agents find its use to supply the metallic ions micro nutrient for the biological growth at very low stabilized concentration.

Second, in some applications the chelating ligand catalyst which has the catalytic activity has been applied as catalyst in the asymmetric synthesis of pharmaceutical substances.

Finally, chelating agents have also been used for human medication. For example, the removal/cleaning with the chelating agent with its chelation termination, including the curing of the lead (Pb) poisoning and other metals with EDTA (ethylenediaminetetraacetic acid) where lead mostly chelated to EDTA, and therefore could be removed from the system.

The publication by H. HAQUE AND A. D. RUSSELL "Effect of Chelating Agents on the Susceptibility of Some Strains of Gram-Negative Bacteria to Some Antibacterial Agents", ANTIMICROBIAL AGENTS AND CHEMOTHERAPY, Aug. 1974, page. 200-206, explains that some chelating agents improve the activity of beta-lactam in the bacterial inhibition of P. aeruginosa strain through the metallic removal from the nutrient medium and some chelating agent could increase the bacterial sensitivity against some antibiotics.

Another journal article published by Kaur P, Vadehra DV, "Effect of certain chelating agents on the antibacterial action of silver nitrate." J Hyg Epidemiol Microbiol Immunol. 1988; 32 (3) :299-306, explains that EDTA and EGTA when applied together with $AgNO_3$ significantly increases the antibacterial action against *Staphylococcus aureus* , where EDTA and EGTA also increase the sensitivity of *Staphylococcus aureus* bacteria that is resistant to $AgNO_3$.

The publication by A. Hinton Jr. and K.D. Ingram, "Comparison of the Antibacterial Activity of Chelating Agents Using the Agar Diffusion Method" International Journal of Poultry Science 9 (11): 1023-1026, 2010 explains that the addition of EDTA chelating agent for the cleaning agent formulation in the poultry treatment could improve the cleaning capability that has the antimicrobial activity helping to reduce poultry corpses contamination.

But until now, there is no publication describing that EDTA itself alone can perform as an antibacterial agent, due to the fact that chelating agents in some cases have the ability to influence simple cell membranes or other organic membranes.

WO 02/081487 A1 discloses tri-substituted derivatives of group V series elements of the periodic table or oxides thereof, which are useful as chelating agents and biocides.

In the US 2003/0055007 patent publication, A1 discloses some lignin sulphonate substance that has the antiviral activity against HIV and also act as an antibacterial agent, but there is no further explanation about the inhibition mechanism of the said substance as the antiviral and antibacterial agent.

Given the above there is a need for the development of new chelating agents and derivatives that are non toxic or less toxic and induce no negative or less negative effects into environment. Such features will significantly increase the application of said chelating agents.

Unexpectedly, the inventors have developed such a new chelating agent that acts alone as antibacterial agent. Specifically, the inventors have developed a series of sulphate chelating agents that confer antibacterial activity by enlarging the membrane porosity of simple cell membranes and other organic membranes. The mechanism for membrane porosity enlargement occurs due to the reaction of the sulphate groups of some sulphate chelating agent with the hydroxyl group ($OH^-$) or amino group ($NH_2^-$) in the peptide bond of modular bond structural of a protein or modular bond structural of a peptidoglycan and their similarities as the main ingredient of the simple cell wall membranes or other organic membranes.

## Brief description of the invention

Moreover, these invented substances have the capability as common chelating agents or substances to open/enlarge the membrane porosity of either simple cell membrane wall or other organic membranes, have potential biocidal property, and it has been discovered that said substances have a very low toxicity against mammal and can also easily be degraded in the environment. All these features make said substances very useful in many applications.

In one embodiment, the present invention provides use of a chelating agent of formula I:

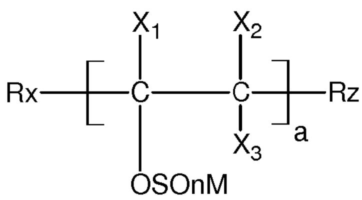

Formula I

wherein:

$X_1$, $X_2$, $X_3$, are selected from: hydroxy, halide, sulphite, sulphate, and sulphonate, where $X_1$, $X_2$, $X_3$, could be similar or different atoms or groups;
Rx, Rz are selected from hydrogen, hydroxy, alkyl $C_{1-20}$, alkylene $C_{1-20}$, alkyl alcohol $C_{1-20}$, aliphatic or branched substituted or unsubstituted, aryl and cycloalkyl substituted or unsubstituted, sulphite, sulphate and sulphonate;
M is hydrogen, or a pharmaceutically acceptable group I, group II, or transition group cation;
n is an integer from 0-3;
a is an integer selected from 1 or more;
and/or a pharmaceutically acceptable salt, solvate, or hydrate of the compound of formula I.

In a preferred embodiment, this invention provides compounds selected from:

- Tetra Hydroxy Ethyl Di Sulphate
- Tetra Hydroxy Butyl 1,2,3,4 Tetra Sulphate
- Tri Hydroxy Butyl 2,3,4-Tri Sulphate
- Tetra Hydroxy Pentyl 2,3,4,5-Tetra Sulphate

In the further embodiment, the preferred substance is where M is Sodium.
In the further embodiment, including its isomer, enantiomer, stereoisomer, salt, solvate, hydrate of Formula I.
In the further embodiment, the preferred substance are:

- Tetrahydroxyethyl disodium 1,2-disulphate
- 1,2,3,4-Tetrahydroxybutyl tetrasodium 1,2,3,4-tetrasulphate
- 1,3-Dihydroxy-2-[hydroxyl(sodium sulphooxy)methyl]propyl dihydrogen 1,3-disulphate
- 1,3,4-Trihydroxy-2-[hydroxyl(sodium sulphooxy)methyl]butyl trihydrogen 1,3,4-trisulphate.

The invention embodiments provide some sulphate chelating agents represented by the above Formula I that has antibacterial activity by enlarging the membrane porosity of simple cells or other organic membranes. The membrane porosity enlargement mechanism for simple cell or other organic membranes occurred due to the reaction of the sulphate groups of some sulphate chelating agent with the hydroxyl group ($OH^-$) or amino group ($NH_2^-$) in the peptide bond of modular bond structural of a protein or modular bond structural of a peptidoglycan and their similarities as the main ingredient of the simple cell wall membranes or other organic membranes.
In another embodiment, this invention provides some sulphate chelating agents represented by the above Formula I that can be used as biocide.
Further embodiment of this invention provides a method of synthesizing a compound according to formula I, said method comprising the steps of:

- reacting formaldehyde, acetaldehyde, formic acid, acetic acid or oxalic acid with sulphuric acid ($H_2SO_4$) or sulphonic acid ($H_2SO_3$) and/or sulfur trioxide ($SO_3$) or sulfur dioxide ($SO_2$).

In the preferred embodiment of this invention, said new substance I can form a complex substance with metal.

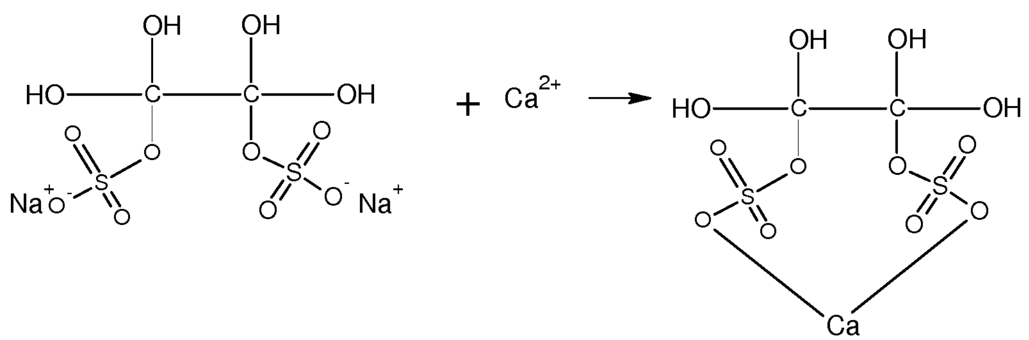

In a further aspect this invention describes a non-therapeutic method of membrane porosity enlargement of simple cells or another organic membranes with some effective amount of substances of invention.
In the preferred embodiment of this invention, the new substances of this invention have the capability to modify

membrane of simple cells or organic membranes by means of enlarging significantly the membrane porosity. While not theoretically fully supported, it is postulated due to sulphate group electronegativity the sulphate chelating agents will stress the hydroxy-hydroxy bond of peptidoglycan and/or hydroxy-amine bond of protein that compose cell membranes or organic membranes. The capability of said substances is proven by means of opening/enlarging membrane porosity of some microorganisms simple cell, such as: bacteria cell, algae, fungi, and similar with some concentration level worked on the membrane modification mechanism from semi permeable into permeable membrane therefore it becomes possible for another ligand insertion into target microorganism and/or simple cell; it is also possible that the microorganism cell wall membranes become totally permeable resulting in the microorganism death due to cell internal lysis or dormant/latent condition of the target microorganism. It is postulated that this mechanism works for almost all of microorganism having cell membranes.

**Brief description of the figures**

Figure 1: Shows the absorbance curve of Tetra Hydroxy Ethyl Di Sulphate Di Sodium (THES)against $NH_3FeSO_4$ volume at pH 7.

Fig 2a : the titration curve shows the chelating capability of THES, where a lower THES concentration is needed in comparison with EDTA.

Fig 3: SEM pictures result of pure $CaCO_3$ crystals with its rhombus structure and $CaCO_3$ crystals after chelated with Tetrahydroxyethyl disodium disulphate (THES) at pH 7.

Figure 4: Transversal section of Staphylococcus aureus single cell (x 35,000), from left to right: Normal cell, in contact with 0.05 % THES, in contact with 1% THES (tetra hydroxy ethyl di sulphate)at pH 7.

Fig 5: Transversal section of E.Coli single cell (x 25,000), from left to right: Normal cell, in contact with 0.05 % THES, in contact with 1% THES (tetrahydroxyethyl disulphate) at pH 7.

Fig 6: Outer view of Staphylococcus aureus cell wall/membranes, from left to right : Normal cell, in contact with 1% THES (tetra hydroxy ethyl di sulphate)at pH 7.

Fig 7: Outer view of E.Coli, cell wall/membranes, from left to right: Normal cell, in contact with 1% THES (tetra hydroxy ethyl di sulphate)at pH 7.

**Detail description of the invention**

In an embodiment, the substance is a compound of formula I:

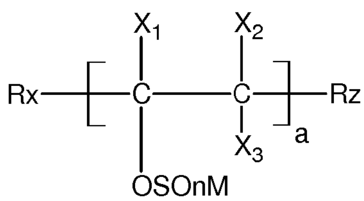

Formula I

wherein:

X may be the same or different and is selected from hydroxy, halide, sulphite, sulphate and sulphonate;

Rx, Rz are selected from hydrogen, hydroxy, alkyl $C_{1-20}$, alkylene $C_{1-20}$, alkyl alcohol $C_{1-20}$, aliphatic or branched substituted or unsubstituted, aryl and cycloalkyl substituted or unsubstituted, sulphite, sulphate and sulphonate;

M is hydrogen, or a pharmaceutically acceptable group I, group II, or transition group cation;

n is an integer from 0-3;

a is integer selected from 1 or more;

and/or a pharmaceutically acceptable salt, solvate, hydrate of the compound of formula I,

and wherein said compound of formula I comprises at least one sulphate group.

In the most preferred embodiment the substance is selected from

- Tetrahydroxyethyl dihydrogen 1,2-disulphate (herein also referred to as Tetra Hydroxy Ethyl Di Sulphate)

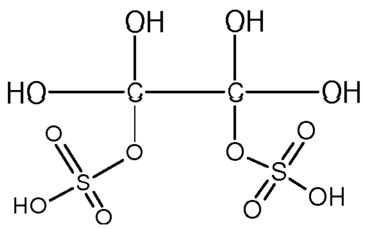

- 1,2,3,4-Tetrahydroxybutyl tetrahydrogen 1,2,3,4-tetrasulphate (herein also referred to as (Tetra Hydroxy Butyl 1,2,3,4 Tetra Sulphate)

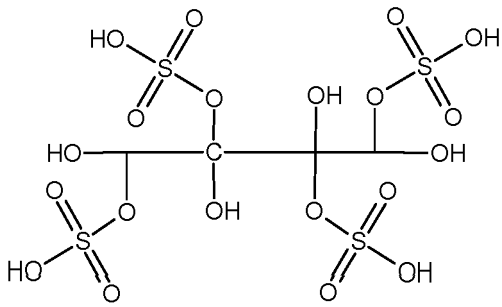

- 1,3-Dihydroxy-2-[hydroxyl(hydrogen sulphooxy)methyl]propyl dihydrogen 1,3-disulphate (herein also referred to as Tri Hydroxy Butyl 2,3,4-Tri Sulphate)

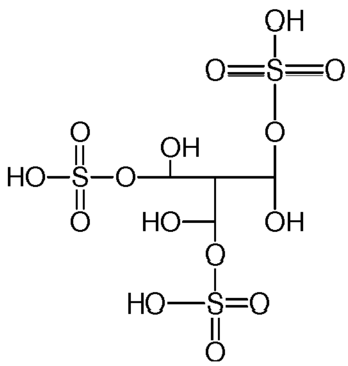

- 1,3,4-Trihydroxy-2-[hydroxyl(hydrogen sulphooxy)methyl]butyl trihydrogen 1,3,4-trisulphate (herein also referred to as Tetra Hydroxy Pentyl 2,3,4,5-Tetra Sulphate)

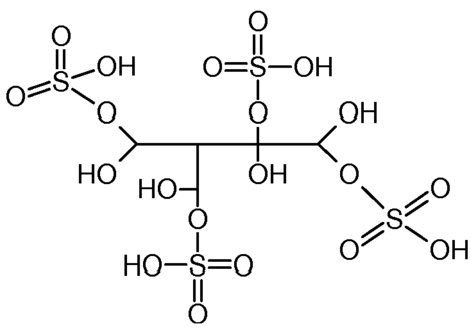

- Ethyl hexahydrogen hexasulphate (herein also referred to as Ethyl Hexa Sulphate acid, orEHS)

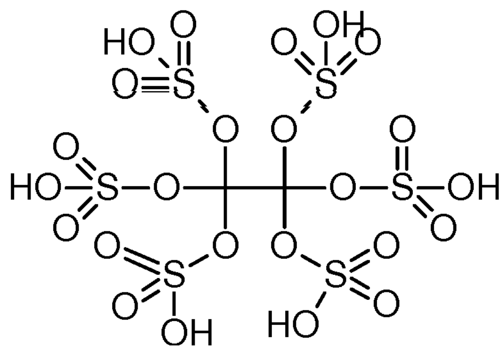

and/or their salt, solvate, hydrate that are pharmaceutically accepted.

In another preferred embodiment the substances are of Formula I, wherein M is Sodium.

In the preferred embodiment the substance is selected from:

- Tetrahydroxyethyl disodium 1,2-disulphate (herein also referred to as Tetra Hydroxy Ethyl Di Sulphate Di Sodium)

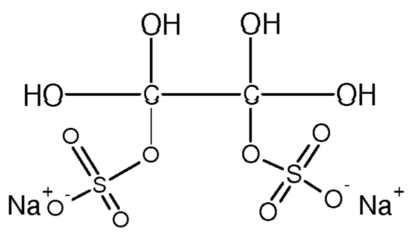

- 1,2,3,4-Tetrahydroxybutyl tetrasodium 1,2,3,4-tetrasulphate (herein also referred to as Tetra Hydroxy Butyl 1,2,3,4 Tetra Sulphate Tetra Sodium)

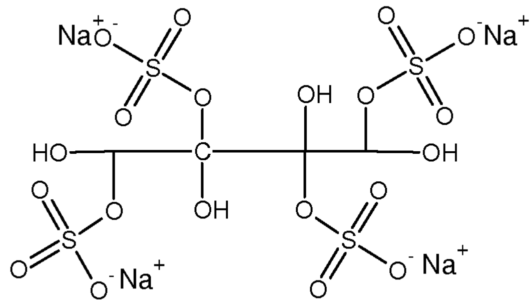

- 1,3-Dihydroxy-2-[hydroxyl(sodium sulphooxy)methyl]propyl disodium 1,3-disulphate (herein also referred to as Tri Hydroxy Butyl 2,3,4-Tri Sulphate Tri Sodium)

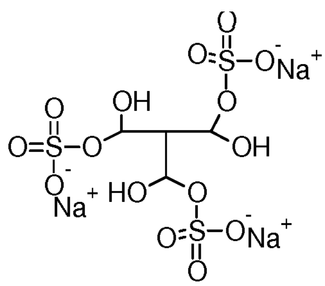

- 1,3,4-Trihydroxy-2-[hydroxyl(sodium sulphooxy)methyl]butyl trisodium 1,3,4-trisulphate (herein also referred to as Tetra Hydroxy Pentyl 2,3,4,5-Tetra Sulphate Tetra Sodium).

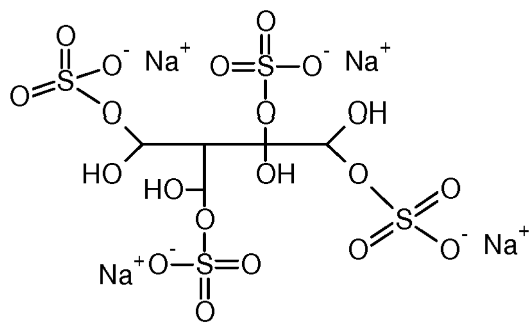

- Ethyl hexasodium hexasulphate- (herein also referred to as Ethyl Hexa Sulphate Hexa Sodium, or EHS-6Na)

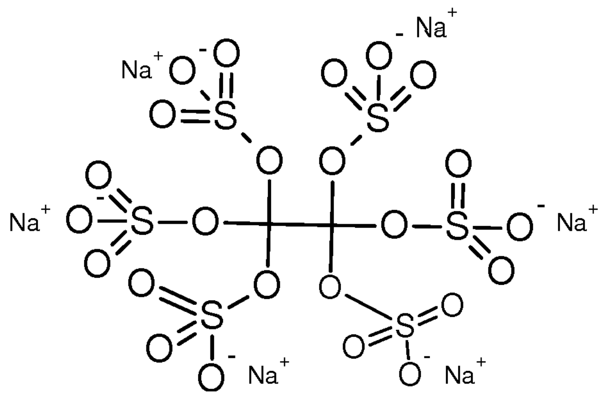

and/or their salt, solvate, hydrate that are pharmaceutically accepted.

Furthermore, the formed alkyl sulphate substance derived from formula I may be polymerized to form a longer chain.

In the preferred embodiment this invention covers a non-therapeutic method of breaking or loosening the hydroxyl-hydroxyl bond of peptidoglycan or hydroxyl-amine bond of protein that compose cell wall membranes or organic membranes by means utilizing some sulphate chelating agent that result on the enlargement of the membrane porosity, therefore the membrane becomes permeable on both of its sides. The capability of the said substance was proven by opening/enlargement simple cell membranes of microorganisms such as: bacteria, algae, fungi, virus, etc. with some concentration level that works on the cell wall membrane modification from semi permeable membrane into more and more permeable, therefore it becomes possible to insert other ligands inside the microorganism or the cell wall membrane becomes totally permeable which results in the microorganism death and/or microorganism dormant condition. It is postulated the said mechanism works for all types of microorganism that has cell membrane.

## Definition

The following terms are used throughout the specification and have the following meanings unless otherwise specified:

"Alkyl" means carbon atom chains having the designated number of carbon atoms which can be either straight chain or branched. Examples of alkyl include but are not limited to, methyl, ethyl, propyl, butyl and isobutyl.

"Alkenyl" means carbon atom chains having the designated number of carbon atoms which can be either straight chain or branched and which contain at least one double bond. The alkenyl compounds may have more than one such double bond and the orientation about each double bond is independently either cis or trans. Examples of alkenyl include, but are not limited to ethenyl, propenyl, butenyl, isobutenyl, pentenyl and hexenyl.

"Alkynyl" means carbon atom chains having the designated number of carbon atoms which can be either straight chain or branched and which contain at least one triple bond.

As used herein the term "aryl" means single, polynucleic conjugated and fused residues of aromatic hydrocarbon or aromatic heterocyclic ring systems, examples of aryl include, but are not limited to phenyl, naphthyl, fluorenyl, pyrenyl, pyridyl and pyrrolyl.

Cycloalkyl termination refers to non-aromatic aliphatic ring moiety of 3-20 mono-cyclic, bicyclic, or polycyclic

carbon atoms. Cycloalkyl can be bicycloalkyl, polycycloalkyl, branched, or spiroalkyl. One or more of the rings might have one or more double bond but no such ring has fully conjugated pi-electron system. Examples, without limitation, of cycloalkyl groups are : cyclopropyl, cyclobutyl, cyclopentyl, cyclopentenyl, cyclohexyl, cyclohexadienyl, adamantyl, cycloheptyl and cycloheptatrienyl.

Sulphite structure could be described with three equal resonance structures. In every resonance structure, the sulphur atom has a double bond with one oxygen atom with zero/neutral charge, and the sulfur atom is singly bonded to the other two oxygen atoms that each has formal charge of -1. It is also found free electrons pair on Sulphur atom, therefore the predicted structure by VSEPR theory is a pyramid trigonal similar to ammonia (NH3).

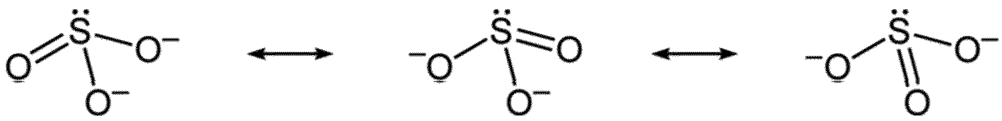

Sulphate termination refers to sulphur as the centered atom surrounded by four equal oxygen atoms in tetrahedral structure. Sulphur atom in its oxidation state +6 while the four oxygen atoms, each in its oxidation state -2. The sulfate ion carries an overall charge of -2 and it is the conjugate base of the bisulfate (or hydrogen sulfate) ion, $HSO_4^-$, which is in turn the conjugate base of $H_2SO_4$, sulfuric acid.

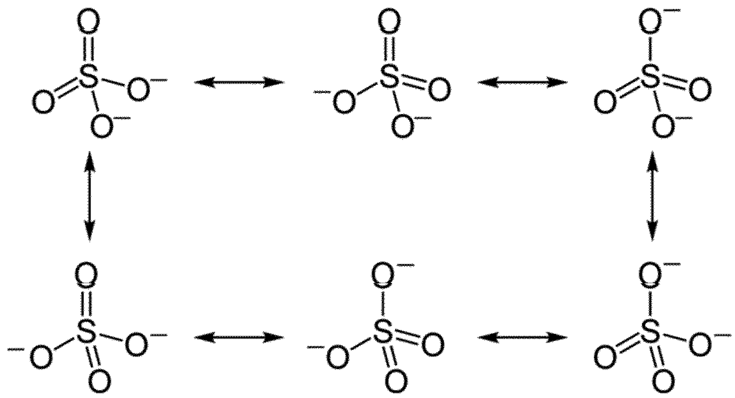

Sulphonate termination refers to an ester of sulphonic acid. An ester with the common formula $ROSO_2R'$ is a sulphonic ester. The common structures of sulphonic ester shown by the structures below:

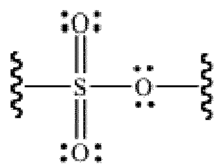

(covalent form)

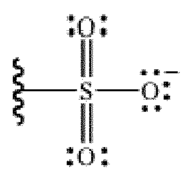

(ionic form)

Pharmaceutically accepted salts refer to alkaline addition types of salts synthesized by adding some alkaline substance to the said substance in this invention. The common pharmaceutically accepted salts are: sodium, potassium, calcium or zinc.

In the specification, the termination of substituted means that a group may be further substituted with one or more groups selected from alkyl, alkenyl, alkynyl, aryl, fluoro, chloro, bromo, hydroxyl, alkoxy, alkenyloxy, aryloxy, acyloxy, amino, alkylamino, dialkylamino, arylamino, thio, alkylthio, arylthio, cyano, nitro, acyl, amido, alkylamido, dialkylamido, carboxyl or two or more substituents may, together with the carbon atoms to which they are attached from a 5 or 6 membered aromatic or non aromatic ring containing 0, 1 or 2 heteroatoms selected from nitrogen, oxygen and sulphur.

A selection of the preferred substances of this invention is:

a. Tetrahydroxyethyl dihydrogen 1,2-disulphate
b. Tetrahydroxyethyl disodium 1,2-disulphate
c. 1,2,3,4-Tetrahydroxybutyl tetrahydrogen 1,2,3,4-tetrasulphate
d. 1,2,3,4-Tetrahydroxybutyl tetrasodium 1,2,3,4-tetrasulphate.
e. 1,3-Dihydroxy-2-[hydroxyl(hydrogen sulphooxy)methyl]propyl dihydrogen 1,3-disulfateButyl
f. 1,3-Dihydroxy-2-[hydroxyl(sodium sulphooxy)methyl]propyl disodium 1,3-disulphate
g. 1,3,4-Trihydroxy-2-[hydroxyl(hydrogen sulphooxy)methyl]butyl trihydrogen 1,3,4-trisulphate
h. 1,3,4-Trihydroxy-2-[hydroxyl(sodium sulphooxy)methyl]butyl trisodium 1,3,4-trisulphate.

**Examples of the synthesis of some substances within this invention:**

**Example 1: Synthesis of Tetra Hydroxy Ethyl Di Sulphate(THES)**

225 g of oxalic acid crystal was diluted in 150 ml of water and the resulted slurry heated to 40-50°C. To this slurry was added dropwise 250 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation until the next 180 minutes with the installation of vertical pipe condenser for reflux purposes to maintain the volume of the liquid until a very clear solution is achieved.

Then the liquid temperature was raised to 90 - 100°C. Evaporation of the solvent followed by crystallization result in formation of the desired substance as an off white powder with the following properties : melting point > 200°C, boiling point > 400°C, specific gravity = 1.86, water solubility 48 %.

**Example 2: Synthesis of Tetra Hydroxy Ethyl Di Sulphate-Di Sodium (THES-2Na)**

90 g of oxalic acid crystal was diluted in 150 ml of water and the resulted slurry heated to 40-50°C. To this slurry was added dropwise 100 ml of 98% $H_2SO_4$. The rate of acid addition was controlled to ensure that the temperature of the slurry heated with agitation until the next 180 minutes with the installation of vertical pipe condenser for reflux was in the range of 90-100°C at atmospheric pressure. Upon completion of $H_2SO_4$ acid addition, the solution is still heated with agitation until the next 180 minutes with the installation of vertical pipe condenser for reflux purposes to maintain the volume of the liquid until a very clear solution is achieved.

To this clear solution was added dropwise a solution of 73 g NaOH flakes in 75 ml of water with the rate of addition being controlled so that the temperature of the solution remained at 50 ± 20°C. Upon completion of addition, the temperature was adjusted slowly to ambient and the solution was held agitated in the ambient temperature for the next 60 minutes.

Evaporation of the solvent followed by crystallization result in formation of the desired substance as an off white powder with the following properties: melting point > 200°C, boiling point > 400°C, specific gravity = 2.2, water solubility 31 %.

**Example 3: Synthesis of Tetra Hydroxy Butyl 1,2,3,4-Tetra Sulphate**

184 g formic acid crystal was diluted in 300 ml of water and the resulted slurry heated to 40-50°C. To this slurry was added dropwise 230 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation for the next 60 minutes, then 8.6 gram of sodium peracetate was added as the catalyst for polymerization process with the installation of vertical pipe condenser during the next 120 minutes reflux until a very clear solution is achieved. Then the temperature was adjusted slowly to ambient and the solution was held agitated in the ambient temperature for the next 60 minutes.

Upon 60 minutes agitation completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tetra Hydroxy Butyl 1,2,3,4 Tetra Sulphate acid crystals, the separated crystals then oven dried at ± 70-80°C until the constant weight reached.

**Example 4: Synthesis of Tetra Hydroxy Butyl 1,2,3,4-Tetra Sulphate-Tetra Sodium**

184 g formic acid crystal was diluted in 300 ml of water and the resulted slurry heated to 40-50°C. To this slurry was added dropwise 230 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation for the next 60 minutes, then 8.6 gram of sodium peracetate was added as the catalyst for polymerization process with the installation of vertical pipe condenser during the next 120 minutes reflux until a very clear solution is achieved. To this clear solution was added dropwise a solution of 170 g NaOH flakes in 200 ml of water with the rate of addition being controlled so that the temperature of the solution remained at 50 ± 20°C. Upon completion of addition, the temperature was adjusted slowly to ambient and the solution was held agitated in the ambient temperature for the next 60 minutes.

Upon 60 minutes agitation completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tetra Hydroxy Butyl 1,2,3,4 Tetra Sulphate Tetra Sodium crystals, the separated crystals then oven dried at ± 70-80°C until the constant weight reached.

**Example 5: Synthesis of Tri Hydroxy Butyl 2,3,4-Tri Sulphate:**

138 g formic acid crystal was diluted in 300 ml of water and the resulted slurry heated to 40-50°C. To this slurry was added dropwise 173 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation for the next 60 minutes, then 8.6 gram of sodium peracetate was added as the catalyst for polymerization process with the installation of vertical pipe condenser during the next 120 minutes reflux until a very clear solution is achieved. Next, prepare methanol solution of 34 g methanol 96 % technical grade in 60 mL aqua; into this solution, 43 g of Caustic soda flakes was added following by 30 minutes agitation, until the salt precipitated perfectly.

Then the said hot liquid was cooling slowly into ambient temperature while still agitated for the next 60 minutes. Separate the precipitated salt through filter to get the clear filtrate.

Upon the filtration completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tri Hydroxy Butyl 2,3,4 Tri Sulphate acid; the separated crystals then oven dried at ± 70-80°C until the constant weight reached.

**Example 6: Synthesis of Tri Hydroxy Butyl 2,3,4-Tri Sulphate-3Sodium**

138 g formic acid crystal was diluted in 300 ml of water and the resulted slurry heated to 40-50°C. To this slurry was added dropwise 173 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation for the next 60 minutes, then 8.6 gram of sodium peracetate was added as the catalyst for polymerization process with the installation of vertical pipe condenser during the next 120 minutes reflux until a very clear solution is achieved. Next, prepare methanol solution of 34 g methanol 96 % technical grade in 60 mL aqua; into this solution, 43 g of Caustic soda flakes was added following by 30 minutes agitation, until the salt precipitated perfectly.

Then the said hot liquid was cooling slowly into ambient temperature while still agitated for the next 60 minutes. Separate the precipitated salt through filter to get the clear filtrate.

To this clear solution was added dropwise a solution of 130 g NaOH flakes in 200 ml of water with the rate of addition being controlled so that the temperature of the solution remained at 50 ± 20°C.

Upon completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tri Hydroxy Butyl 2,3,4 Tri Sulphate-3Sodium crystals; the separated crystals then oven dried at ± 70-80°C until the constant weight reached.

**Example 7: Synthesis of Tetra Hydroxy Pentyl 2,3,4,5 Tetra Sulphate**

184 g formic acid crystal was diluted in 300 ml of water and the resulted slurry heated to 40-50°C. To this slurry was added dropwise 230 ml of 98% $H_2SO_4$. The rate of acid addition was controlled to ensure that the temperature of the slurry was in the range of 90-100°C at atmospheric pressure. Upon completion of $H_2SO_4$ acid addition, the solution

is still heated with agitation for the next 60 minutes, then 8.6 gram of sodium peracetate was added as the catalyst for polymerization process with the installation of vertical pipe condenser during the next 120 minutes reflux until a very clear solution is achieved. Next, prepare methanol solution of 34 g methanol 96 % technical grade in 60 mL aqua; into this solution, 43 g of Caustic soda flakes was added following by 30 minutes agitation, until the salt precipitated perfectly.

Then the said hot liquid was cooling slowly into ambient temperature while still agitated for the next 60 minutes. Separate the precipitated salt through filter to get the clear filtrate.

Upon the filtration completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tetra Hydroxy Pentyl 2,3,4,5 Tetra Sulphate acid; the separated crystals then oven dried at ± 70-80°C until the constant weight reached.

**Example 8: Synthesis of Tetra Hydroxy Pentyl 2,3,4,5 Tetra Sulfat-4Sodium**

184 g formic acid crystal was diluted in 300 ml of water and the resulted slurry heated to 40-50°C. To this slurry was added dropwise 230 ml of 98% $H_2SO_4$. The rate of acid addition was controlled to ensure that the temperature of the slurry was in the range of 90-100°C at atmospheric pressure. Upon completion of $H_2SO_4$ acid addition, the solution is still heated with agitation for the next 60 minutes, then 8.6 gram of sodium peracetate was added as the catalyst for polymerization process with the installation of vertical pipe condenser during the next 120 minutes reflux until a very clear solution is achieved.

Next, prepare methanol solution of 34 g methanol 96 % technical grade in 60 mL aqua; into this solution, 43 g of Caustic soda flakes was added following by 30 minutes agitation, until the salt precipitated perfectly.

Then the said hot liquid was cooling slowly into ambient temperature while still agitated for the next 60 minutes. Separate the precipitated salt through filter to get the clear filtrate.

To this clear solution was added dropwise a solution of 170 g NaOH flakes in 200 ml of water with the rate of addition being controlled so that the temperature of the solution remained at 50 ± 20°C.

Upon completion, the condenser was released, then the solution is boiled for around 2 hours to evaporate the water until 35 - 40 % of its original volume evaporated. The resulting solution then undergoes slow cooling into ambient temperature and cooling further into ± 10 °C by *ice water bath.* The resulting suspension then is filtrated to separate the Tetra Hydroxy Pentyl 2,3,4,5 Tetra Sulphate-4Sodium; the separated crystals then oven dried at ± 70-80°C until the constant weight reached.

**Example 9: Synthesis of Ethyl Hexa Sulphate (EHS)**

75 g of oxalic acid crystal was diluted in 75 ml of water and the resulted slurry heated to 40-50°C. To this slurry was added dropwise 250 ml of 98% $H_2SO_4$. The rate of acid addition was controlled to ensure that the temperature of the slurry heated with agitation until the next 180 minutes with the installation of vertical pipe condenser for reflux was in the range of 90-100°C at atmospheric pressure. Upon completion of $H_2SO_4$ acid addition, the solution is still heated with agitation until the next 120 minutes with the installation of vertical pipe condenser for reflux purposes to maintain the volume of the liquid until a very clear solution is achieved; then the vertical pipe condenser was released, the resulting solution is still heated with another 30 minutes agitation to ensure complete and homogenous sulphatation reaction.

Then the liquid temperature was raised to 100 - 105°C. Evaporate half of the solvent volume followed by crystallization result in formation of the desired substance as an off white powder with the following properties : melting point > 210°C, boiling point > 400°C, specific gravity = 1.87, water solubility 48 % and very hygroscopic.

**Example 10: Synthesis of Ethyl Hexa Sulphate-Hexa Sodium (EHS-6Na)**

75 g of oxalic acid crystal was diluted in 75 ml of water and the resulted slurry heated to 40-50°C. To this slurry was added dropwise 250 ml of 98% $H_2SO_4$. The rate of acid addition was controlled to ensure that the temperature of the slurry heated with agitation until the next 180 minutes with the installation of vertical pipe condenser for reflux was in the range of 90-100°C at atmospheric pressure. Upon completion of $H_2SO_4$ acid addition, the solution is still heated with agitation until the next 120 minutes with the installation of vertical pipe condenser for reflux purposes to maintain the volume of the liquid until a very clear solution is achieved; then to this clear solution was added dropwise a solution of 185 g NaOH flakes in 190 ml of water with the rate of addition being controlled so that the temperature of the solution remained at 50 ± 20°C within one hours time. Then the vertical pipe condenser was released, the resulting solution is still heated with another 30 minutes agitation to ensure complete and homogenous neutralization reaction.

Then the liquid temperature was raised to 100 - 105°C. Evaporate half of the solvent volume followed by crystallization result in formation of the desired substance as an off white powder with the following properties : melting

point > 360°C, boiling point > 400°C, specific gravity = 2.21, water solubility 29 % and hygroscopic.

**Analytical method:**

**Analytical method to determine Tetra Hydroxy Ethyl di Sulphate-di Sodium (THES-2Na), utilizing Iron's chelating agent**

Test Principle :

Active matter of Tetra Hydroxy Ethyl Di Sulphate-Di Sodium (THES-2Na) was determined by chelating reaction of residual metallic ion with several anionic surfactants that prevent this anionic surfactants dissolved in the solution result in the turbid solution measured by its turbidity and/or absorbance spectrophotometer.

Materials and Equipment:

*Materials:*

Reagent grade $NH_3FeSO_4.2H_2O$, reagent grade Sodium Lauryl Ether sulphate (Na-SLES), Caustic Soda flakes, water (aqua).

*Equipment:*

Visible Spectrophotometer, cuvette, erlenmeyer, beaker glass, pipette, scale weight, magnetic stirrer.

*Method:*

I. Absorbance:

1) Add 2 gr Na-SLES into 100 $cm^3$ aqua, stir with magnetic stirrer for 15 minutes until clear solution (1) reached.
2) Add on 1,0 $cm^3$ of tested chelating agent (THES-2Na), stir with magnetic stirrer for 15 minutes until homogenous solution(2).
3) Adjust the pH of the solution above into 7 with Caustic Soda flakes.
4) Prepare 50 $cm^3$ 0,1 M NH3FeSO4 solution in the 100 $cm^3$ beaker glass.
5) Fill the cuvette with the solution (2), record the absorbance at 450 nm.
6) Titration starts with the addition of 1,0 $cm^3$ 0,1 M NH3FeSO4 solution into solution(2), stir with magnetic stirrer for 2 minutes until homogenous solution(3).
7) Fill the cuvette with the solution(3), record the absorbance at 450 nm.
8) Repeat step 5 and 6 until end point of 0,1 M NH3FeSO4 reached.

End point and calculation

I. *Absorbance method*:

1) Absorbance reading will show significant increases, until some points, then the reading will decrease.
2) When this absorbance reading suddenly jump from the increasing trend into decreasing trend, the end point of the titration has reached.
3) Continue the NH3FeSO4 solution addition by another 2 or 3 $cm^3$ volume to get the smooth curve and/or representative data for accurate interpolation of the end point volume.
4) Draw the absorbance curve versus titrant volume addition then determine the end point volume by this curve or using all absorbance and NH3FeSO4 volume addition through Lagrance interpolation formula to get the end point volume.
5) Calculate the concentration or active matter of Tetra Hydroxy Ethyl Di Sulphate Di Sodium(THES-2Na)using the formula below :

THES 2Na content = (V ep x M NH3FeSO4 x MW THES 2Na x 3,9)/(V sample x sample SG x 1,000)

**where :**

| | |
|---|---|
| V ep | : end point volume in $cm^3$ |
| M NH3FeSO4 | : molarity NH3FeSO4: 0,1 M |
| BM THES 2Na | : molecule weight THES 2Na: 330 |
| V sample | : sample volume of tested chelant in $cm^3$ |

**Example :**

Take 1,0 $cm^3$ of 35% tetrahydroxyethyl disodium 1,2-disulphate (THES-2Na)solution as sample, density of 1,1 g/$cm^3$. Then add on Na-SLES, titrate with 0,1 M NH3FeSO4; the absorbance record shown in the Table 1 below:

**Table 1**

| No | ml titer NH3FeSO4 | Absorbance 450 nm |
|---|---|---|
| 1 | 0 | 0,001 |
| 2 | 1 | 0,011 |
| 3 | 2 | 0,025 |
| 4 | 3 | 0,042 |
| 5 | 4 | 0,156 |
| 6 | 5 | 0,241 |
| 7 | 6 | 0,32 |
| 8 | 7 | 0,355 |
| 9 | 8 | 0,385 |
| 10 | 9 | 0,397 |
| 11 | 10 | 0,404 |
| 12 | 11 | 0,41 |
| 13 | 12 | 0,411 |
| 14 | 13 | 0,414 |
| 15 | 14 | 0,413 |
| 16 | 15 | 0,412 |

From Table 1 above, the absorbance curve versus volume NH3FeSO4 trend is shown in Figure 1.

From Figure 1, the end point volume is 3,5 ml, Molarity NH3FeSO4 is 0,1 M, MW THES-2Na is 330 and sample volume of 1,0 $cm^3$ with SG 1.1 :
THES-2Na concentration = 3,2 $cm^3$x 0,1 M x 330 x 3,9/(1,0 x 1,15 x 1,000) = 0,358 = 35,8 % of chelating agent.

**Characteristics of this new invention substance**

These substances of this new invention are preferred to have the characteristics as below:
a. Tetra Hydroxy Ethyl Di Sulphate (THES)

| | |
|---|---|
| Molecular weight | 286 |
| Crystal density (g/$cm^3$) | 1.865 |
| Solubility @ 25 °C (%) | 48.0 |

(continued)

| | |
|---|---|
| pH of 30 % solution | < 0.5 |
| Melting point (°C) | 218 |
| Boiling point (°C) | > 400 |
| Colour | Off white |
| Crystal characteristic | Hygroscopic |

b. Tetra Hydroxy Ethyl Di Sulphate Di Sodium

| | |
|---|---|
| Molecular weight | 330 |
| Crystal density (g/cm$^3$) | 2.215 |
| Solubility @ 25 °C (%) | 31.5 |
| pH of 30 % solution | 0.5-1.5 |
| Melting point (°C) | 287 |
| Boiling point (°C) | >400 |
| Colour | Transparent |
| Crystal characteristic | Slightly hygroscopic |

c. Tetra Hydroxy Butyl 1,2,3,4 Tetra Sulphate

| | |
|---|---|
| Molecular weight | 506 |
| Crystal density (g/cm$^3$) | 2.28 |
| Solubility @ 25 °C (%) | 39.6 |
| pH of 30 % solution | <0.0 |
| Melting point (°C) | 242 |
| Boiling point (°C) | >400 |
| Colour | White brownies |
| Crystal characteristic | Very hygroscopic |

d. Tetra Hydroxy Butyl 1,2,3,4 Tetra Sulphate Tetra Sodium

| | |
|---|---|
| Molecular weight | 594 |
| Crystal density (g/cm$^3$) | 2.978 |
| Solubility @ 25 °C (%) | 27.3 |
| pH of 30 % solution | 0.0-1.0 |
| Melting point (°C) | 288 |
| Boiling point (°C) | >400 |
| Colour | Transparent |
| Crystal characteristic | hygroscopic |

e.1,3,4-Trihydroxy-2-[hydroxyl(hydrogen sulphooxy)methyl]butyl trihydrogen 1,3, 4-trisulphate

| | |
|---|---|
| Molecular weight | 393 |
| Crystal density (g/cm$^3$) | 1.97 |
| Solubility @ 25 °C (%) | 56.7 |
| pH of 30 % solution | <0.0 |
| Melting point (°C) | 191 |
| Boiling point (°C) | >400 |
| Colour | White brownies |
| Crystal characteristic | Very hygroscopic |

f. 1,3-Dihydroxy-2-[hydroxyl(sodium sulphooxy)methyl]propyl disodium 1, 3-disulphate

| | |
|---|---|
| Molecular weight | 459 |
| Crystal density (g/cm$^3$) | 2.35 |
| Solubility @ 25 °C (%) | 36.2 |
| pH of 30 % solution | 0.0-1.0 |
| Melting point (°C) | 242 |
| Boiling point (°C) | >400 |
| Colour | Off white |
| Crystal characteristic | hygroscopic |

g.1,3,4-Trihydroxy-2-[hydroxyl(hydrogen sulphooxy)methyl]butyl trihydrogen 1,3, 4-trisulphate

| | |
|---|---|
| Molecular weight | 519 |
| Crystal density (g/cm$^3$) | 2.07 |
| Solubility @ 25 °C (%) | 61.2 |
| pH of 30 % solution | <0 |
| Melting point (°C) | 211 |
| Boiling point (°C) | >400 |
| Colour | White brownies |
| Crystal characteristic | Very hygroscopic |

h. 1,3,4-Trihydroxy-2-[hydroxyl(sodium sulphooxy)methyl]butyl trisodium 1,3, 4-trisulphate

| | |
|---|---|
| Molecular weight | 607 |
| Crystal density (g/cm$^3$) | 2.63 |
| Solubility @ 25 °C (%) | 40.1 |
| pH of 30 % solution | 0.0-0.5 |
| Melting point (°C) | 247 |
| Boiling point (°C) | >400 |
| Colour | Off white |
| Crystal characteristic | hygroscopic |

i. Ethyl hexahydrogen hexasulphate(EHS)

| | |
|---|---|
| Molecular weight | 606 |
| Crystal density (g/cm$^3$) | 1,87 |
| Solubility @ 25 °C (%) | 48% |
| pH of 30 % solution | 0.0 |
| Melting point (°C) | >210°C |
| Boiling point (°C) | >400°C |
| Colour | Off white |
| Crystal characteristic | hygroscopic |

j. Ethyl hexasodium hexasulphate (EHS-6Na)

| | |
|---|---|
| Molecular weight | 738 |
| Crystal density (g/cm$^3$) | 1,87 |
| Solubility @ 25 °C (%) | 48% |
| pH of 30 % solution | 0.0-0.5 |
| Melting point (°C) | >210°C |
| Boiling point (°C) | >400°C |
| Colour | Off white |
| Crystal characteristic | hygroscopic |

Ability to enlarge the simple cell membrane

The substances of this invention were found to have the ability to modify the cell membrane or organic membrane by means enlarging/loosening membrane porosity significantly. By not totally theoretically supported, it was postulated due to the sulphate group electronegativity in the said substance as the chelating agent will loosen the hydroxy-hydroxy bond of a peptidoglycan or hydroxy-amino bond of a protein that compose the membrane cell or organic membrane. The ability of these substances to enlarge/loosen the membrane porosity were proven by opening/enlarging the microorganism cell membrane, ie: bacteria, algae, fungi, virus, etc with such concentration level that works on the cell wall membrane modification from the semi permeable into more permeable until it is possible to insert other ligands inside the microorganism or the cell wall membrane becomes totally permeable which results in the microorganism death or its latent position. It has been thought that the said mechanism works for all microorganism that has the cell membrane. The said ability to modify the simple cell membrane permeability will be shown by the mechanism and testing data below:

Method:

The ability of Tetra Hydroxy Ethyl Di Sulphate-Di Sodium (THES) as a Sulphate chelating agent to open/loosen the cell membrane was tested against two types of bacteria, ie: Staphylococcus aureus (gram positive) that has two cell membranes (the outer membrane as the outer cell wall and the inner membrane) and E. Coli (gram negative) that has three cell membranes. First, the Minimum Inhibitory Concentration (MIC) of each bacteria was determined, then the bacteria membrane alteration after contacting with THES was observed by Electron Microscope (TEM and SEM) with the THES concentration range below and above MIC. Antibacterial activity was tested using disc-diffussion method by checking the clear zone from the paper disc inhibition that contains THES. The growth curve were made by means examining the bacterial culture on the broth media.

Materials and equipment:

*Materials* :

Difco brand nutrient broth, distilled water (aqua destillata), THES (tetra hydroxy ethyl di sulphate di sodium) as the tested substance, glutaraldehyde, paraformaldehyde, bovine serum albumin (BSA), buffer phosphate pH 7, NaCl 0.9 %, white LR resin, Toluidine blue, Sodium citrate trihydrate, NaOH 0,1N, uranyl acetate, lead nitrate ($Pb(NO_3)_2$), paraffin, transparent capsule, glass cutter, grid, collodion, gold powder.

Equipment :

Incubator, autoclave, petri dish, ose, spectrophotometer (Bausch&Lomb), vibrating incubator, glassware, ultra microtom, knife maker, visible microscope, grid pad, transmission electron microscope (Philips), scanning electron microscope (Hitachi).

**Table 2 : Result of Biocide effication plate test of Tetra Hydroxy Ethyl Di Sulphate-Di Sodium (THES-Di Sodium)**

| No | Biocide dosing | Total Plate Count (Colony/ml) | Killing efficiency |
|---|---|---|---|
| | **Total Plate Count Microorganism** | | |
| 1 | Blanco/Control | 2400 | 0 |
| 2 | 0.05 % THES-di Sodium | 132 | 94.5 |
| 3 | 0.1 % THES-di Sodium | 115 | 95.2 |
| 4 | 0.5 % THES-di Sodium | 101 | 95.8 |
| 5 | 1.0 % THES-di Sodium | 80 | 96.7 |
| | | | |
| | ***E.Coli*** | | |
| 1 | Blanco | 3900 | 0 |
| 2 | 0.025 % THES-di Sodium | 2010 | 48.5 |
| 3 | 0.05 % THES-di Sodium | 1150 | 70.5 |
| 4 | 0.1 % THES-di Sodium | 1050 | 73.1 |
| 5 | 0.5 % THES-di Sodium | 970 | 75.1 |
| 6 | 1.0 % THES-di Sodium | 720 | 81.5 |
| 7 | 5.0 % THES-di Sodium | 630 | 83.8 |
| | | | |
| | ***Staphylococcus Aerus*** | | |
| 1 | Blanco | 980 | 0 |
| 2 | 0.025 % THES-di Sodium | 296 | 69.8 |

*Test on Microorganism:*

*Staphylococcus aureus* ATCC 6538, *Escherichia coli* ATCC 9637 were supplied from Chemotherapy Laboratory of Pharmaceutical Department, Bandung Institute of Technology (ITB) .

Method:

1. Bacteria preparation

The testing bacteria were growth on the aqueous medium and incubated for 24 hours at 37°C.

2. Tested Biocide preparation

The tested Biocide was diluted in distilled water at various gradually decreased concentration. This gradual concentration variation is necessary to determine MIC (Minimum Inhibitory Concentration) and the bacteriostatic/bactericidal properties of the tested Biocide.

3. Determination of Killing method (bacteriostatic and bactericidal properties)

The testing was done on the aqueous medium containing the tested substance with the concentration 5, 10 and 20%. The turbidity of the cultured medium then will be measured every 30 minutes until 270 minutes with spectrophotometer.

4. Preparation of microbe cells for electron microscope examination

*Staphylococcus aureus* and Escherichia coli were grown on the aqueous medium within 24 hours, then mixed with tetra hydroxy ethyl di sulphate di sodium (THES) with the final concentration 0,6; 5 and 10 %. This system mixture then was placed under vibrating incubator for several hours. Microbe cells then were separated and washed, first with NaCl 0,9 % then with distilled water. Cells then were utilized for the preparation of electron microscope testing.

5. Preparation for Scanning Electron Microscope (SEM) examination

Bacteria cells were suspended into 4% collodion. The said suspensions then were fixed on some metal disc and were coated with the gold powder. Then were examined with electron microscope and pictured.

6. Preparation for Transmittance Electron Microscope (TEM) examination

a) The prepared microbes in (5) were fixed into a mixture of formaldehyde 2% and glutaraldehyde 0.5% for 2 hours at 4°C.
b) The above mixture then was three times washed with pH 7 phosphate buffer, then was diluted into bovine serum albumin (BSA) 1/5 for embedded preparation, while leaving glutaraldehyde hardening at room temperature.
c) After leaving dehydrated in alcohol, the white LR resin then will be infiltrated.
d) Embedded processes were done at white LR resin following by polymerization process at 60°C for 48 hours time. The polymerization product as hardened capsules then is ready to be sliced.
e) Making of semi thin and ultra thin slices. Slices of microbe sample were made by utilizing automatic ultra microtom. To get the precise and accurate slices of sample, semi thin slices were made and coloured with Toluidine blue then were examined under the visible microscope. When the right slices were obtained, ultra thin slices were prepared and were made contrast with uranyl acetate and lead citrate. The dried ultra thin slices then can be examined with Transmittance Electron Microscope.

Testing results

<u>Minimum inhibitory Concentration (MIC)</u>

MIC of tetra hydroxy ethyl di sulphate di sodium against *E.coli* and *S.aureus* are shown in Table 3.

**Table 3 : Antibacterial activity of Tetra Hydroxy Ethyl Di Sulphate-Di Sodium (THES-Di Sodium)**

| **No** | **Microorganism** | **Minimum Inhibitory Concentration (MIC) (%)** |
|---|---|---|
| | **Gram Positive Bacteria** | |
| 1 | *Bacillus Subtilis* ATCC 6633 | 0,234 |
| 2 | *Staphylococcus Aerus* ATCC 6538 | 0,234 |
| 3 | *Staphylococcus Epidermidis* ATCC 12228 | 0,234 |

(continued)

| No | Microorganism | Minimum Inhibitory Concentration (MIC) (%) |
|---|---|---|
| | **Gram Positive Bacteria** | |
| | | |
| | **Gram Negative Bacteria** | |
| 1 | *Acinetobacter Anitratus* | 0,115 |
| 2 | *Escherichia Coli* ATCC 25922 | 0,234 |
| 3 | *Pseudomonas Aeruginosa* ATCC 27853 | 0,115 |
| 4 | *Klebsiella Pneumoniae* | 0,234 |
| 5 | *Salmonella Typhii* | 0,115 |
| | | |
| | **Fungi** | |
| 1 | *Aspergillus Niger* | 0,937 |
| 2 | *Candida Albicans* | 0,469 |

The test done on the pH range of 7-7,5 with the sample of Tetra Hydroxy Ethyl Di Sulphate-Di Sodium (THES-Di Sodium) 30% in aqueous solution.

From the transversal section (TEM) of Staphylococcus aureus cell, can be observed between normal cell that still contains cytoplasm and other organic substance (dark sight)and the partly empty cells that has already lost its cytoplasm liquid due to lysis out of cell wall membrane (transparent sight), in fact the E.Coli cell that has more layers cell wall membranes also experienced the said internal cell liquid lysis out of its cell wall membrane layers, although the MIC of E.Coli cell was slightly higher than Staphylococcus aureus cell.

From the outer surface of bacteria's cell wall observation through Scanning Electron Microscope (SEM), it was shown that the outer cell wall membrane of bacteria looked wrinkled/corrugated due to the wider cell wall surface compared to normal cell wall for both Staphylococcus aureus and E.Coli.

## Claims

1. Non-therapeutic use of 1,3-dihydroxy-2-[hydroxyl(hydrogen sulphooxy)methyl]propyl dihydrogen 1,3-disulfate, 1,3,4-trihydroxy-2-[hydroxyl(hydrogen sulphooxy)methyl]butyl trihydrogen 1,3,4-trisulfate, or a compound of formula I below as a microorganism cell wall membrane loosening or opening agent to enlarge the porosity of the membrane,

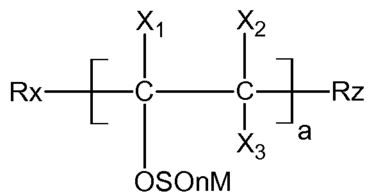

Formula I

wherein:

$X_1$, $X_2$, $X_3$, are selected from: hydroxy, halide, sulphite, sulphate, and sulphonate, where $X_1$, $X_2$, $X_3$, could be similar or different atoms or groups;
Rx, Rz are selected from: hydrogen, hydroxy, alkyl $C_{1-20}$, alkylene $C_{1-20}$, alkyl alcohol $C_{1-20}$, aliphatic or branched substituted or unsubstituted, aryl, cycloalkyl substituted or unsubstituted, sulphite, sulphate, and sulphonate;
M: is hydrogen, or a group I, group II, transition group cation that is pharmaceutically acceptable.
n is an integer from 0-3

a is an integer selected from 1 or more
and/or their salt, solvate, hydrate that is pharmaceutically acceptable, and wherein said compound of formula I comprises at least one sulphate group.

**2.** Non-therapeutic use of 1,3-dihydroxy-2-[hydroxyl(hydrogen sulphooxy)methyl]propyl dihydrogen 1,3-disulfate, 1,3,4-trihydroxy-2-[hydroxyl(hydrogen sulphooxy)methyl]butyl trihydrogen 1,3,4-trisulfate, or the compound of formula I as defined in claim 1 as biocide.

**3.** Non-therapeutic use of 1,3-dihydroxy-2-[hydroxyl(hydrogen sulphooxy)methyl]propyl dihydrogen 1,3-disulfate, 1,3,4-trihydroxy-2-[hydroxyl(hydrogen sulphooxy)methyl]butyl trihydrogen 1,3,4-trisulfate, or the compound of formula I as defined in claim 1 as chelating agent.

**4.** Non-therapeutic use according to any one of claims 1-3, wherein the compound according to formula I is selected out of:

- Tetrahydroxyethyl dihydrogen 1,2-disulphate
- 1,2,3,4-Tetrahydroxybutyl tetrahydrogen 1,2,3,4-tetrasulphate
- Ethyl hexahydrogen hexasulphate

**5.** Non-therapeutic use according to any one of claims 1-3, wherein M is sodium.

**6.** Non-therapeutic use according to any one of claims 1-5, wherein the compound according to formula I is selected out of:

- Tetrahydroxyethyl disodium 1,2-disulphate
- 1,2,3,4-tetrahydroxybutyl tetrasodium 1,2,3,4tetrasulphate
- Ethyl hexasodium hexasulphate (EHS-6Na).

**7.** 1,3-dihydroxy-2-[hydroxyl(hydrogen sulphooxy)methyl]propyl dihydrogen 1,3-disulfate, 1,3,4-trihydroxy-2-[hydroxyl(hydrogen sulphooxy)methyl]butyl trihydrogen 1,3,4-trisulfate, or a compound of formula I below as a microorganism cell wall membrane loosening or opening agent to enlarge the porosity of the membrane, for use in the treatment of a bacterial, viral, fungal or algal infection,

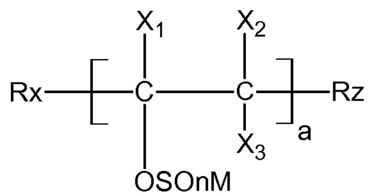

Formula I

wherein:

$X_1$, $X_2$, $X_3$, are selected from: hydroxy, halide, sulphite, sulphate, and sulphonate, where $X_1$, $X_2$, $X_3$, could be similar or different atoms or groups;
Rx, Rz are selected from: hydrogen, hydroxy, alkyl $C_{1-20}$, alkylene $C_{1-20}$, alkyl alcohol $C_{1-20}$, aliphatic or branched substituted or unsubstituted, aryl, cycloalkyl substituted or unsubstituted, sulphite, sulphate, and sulphonate;
M: is hydrogen, or a group I, group II, transition group cation that is pharmaceutically acceptable.
n is an integer from 0-3
a is an integer selected from 1 or more
and/or their salt, solvate, hydrate that is pharmaceutically acceptable, and wherein said compound of formula I comprises at least one sulphate group.

**8.** 1,3-dihydroxy-2-[hydroxyl(hydrogen sulphooxy)methyl]propyl dihydrogen 1,3-disulfate, 1,3,4-trihydroxy-2-[hydroxyl(hydrogen sulphooxy)methyl]butyl trihydrogen 1,3,4-trisulfate, or the compound of formula I as defined in claim 7 for use according to claim 7 as biocide.

9. Compound for use according to any one of claims 7-8, wherein the compound according to formula I is selected out of:

- Tetrahydroxyethyl dihydrogen 1,2-disulphate
- 1,2,3,4-Tetrahydroxybutyl tetrahydrogen 1,2,3,4-tetrasulphate
- Ethyl hexahydrogen hexasulphate

10. Compound for use according to any one of claims 7-8, wherein M is sodium.

11. Compound for use according to any one of claims 7-10, wherein the compound according to formula I is selected out of:

- Tetrahydroxyethyl disodium 1,2-disulphate
- 1,2,3,4-tetrahydroxybutyl tetrasodium 1,2,3,4tetrasulphate
- Ethyl hexasodium hexasulphate (EHS-6Na).

12. A compound that is selected from:

- Tetrahydroxyethyl dihydrogen 1,2-disulphate
- 1,2,3,4-Tetrahydroxybutyl tetrahydrogen 1,2,3,4-tetrasulphate
- 1,3-Dihydroxy-2-[hydroxyl(hydrogen sulphooxy)methyl]propyl dihydrogen 1,3-disulfate
- 1,3,4-Trihydroxy-2-[hydroxyl(hydrogen sulphooxy)methyl]butyl trihydrogen 1,3,4-trisulfate
- Ethyl hexahydrogen hexasulphate

13. A compound that is selected from:

- Tetrahydroxyethyl disodium 1,2-disulphate
- 1,2,3,4-Tetrahydroxybutyl tetrasodium 1,2,3,4-tetrasulphate
- 1,3-Dihydroxy-2-[hydroxyl(sodium sulphooxy)methyl]propyl disodium 1,3-disulfate
- 1,3,4-Trihydroxy-2-[hydroxyl(sodium sulphooxy)methyl]butyl trisodium 1,3,4-trisulfate.
- Ethyl hexasodium hexasulphate (EHS-6Na).

14. Synthesis of the compound according to formula I as defined in claim 1 or according to any of claims 12 or 13, that comprises the step of:

- reacting formaldehyde, acetaldehyde, formic acid, acetic acid or oxalic acid with sulphuric acid ($H_2SO_4$) or sulphonic acid ($H_2SO_3$) and/or sulfur trioxide ($SO_3$) or sulfur dioxide ($SO_2$).

15. A non-therapeutic method to break or loosen the hydroxy-hydroxy bond on peptidoglycan or hydroxyl-amide bond on protein comprised in a microorganism cell wall membrane or another organic membrane resulting in an enlargement of the membrane porosity by treating said membrane with the compound according to formula I as defined in claim 1, wherein said compound enlarges the membrane porosity that turns the membrane into totally permeable on both of its sides.

## Patentansprüche

1. Nicht-therapeutische Verwendung von 1,3-Dihydroxy-2-[hydroxyl(hydrogen-sulfooxy)methyl]propyl-dihydrogen-1,3-Disulfat, 1,3,4-Trihydroxy-2-[hydroxyl(hydrogen-sulfooxy)methyl]butyl-trihydrogen-1,3,4-Trisulfat, oder einer Verbindung der nachstehenden Formel I als Lockerungs- oder Öffnungsmittel für eine Zellwand-Membran von Mikroorganismen, um die Porosität der Membran zu vergrößern,

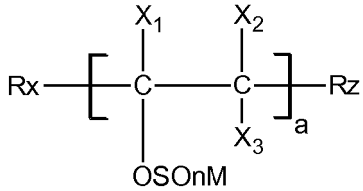

Formel I

wobei:

$X_1$, $X_2$, $X_3$ ausgewählt sind aus: Hydroxy, Halogenid, Sulfit, Sulfat, und Sulfonat, wobei $X_1$, $X_2$, $X_3$ gleiche oder verschiedene Atome oder Gruppen sein können;
Rx, Rz ausgewählt sind aus: Wasserstoff, Hydroxy, Alkyl $C_{1-20}$, Alkylen $C_{1-20}$, Alkylalkohol $C_{1-20}$ aliphatisch oder verzweigt substituiert oder unsubstituiert, Aryl, Cycloalkyl substituiert oder unsubstituiert, Sulfit, Sulfat, und Sulfonat;
M: Wasserstoff oder ein Gruppe I, Gruppe II, Übergangsgruppenkation ist, das pharmazeutisch verträglich ist.
n eine ganze Zahl von 0-3 ist
a eine ganze Zahl, ausgewählt aus 1 oder mehr,
und/oder deren Salz, Solvat, Hydrat ist, das pharmazeutisch verträglich ist, und wobei die Verbindung der Formel I mindestens eine Sulfatgruppe umfasst.

**2.** Nicht-therapeutische Verwendung von 1,3-Dihydroxy-2-[hydroxyl(hydrogen-sulfooxy)methyl]propyl-dihydrogen-1,3-Disulfat, 1,3,4-Trihydroxy-2-[hydroxyl(hydrogen-sulfooxy)methyl]butyl-trihydrogen-1,3,4-Trisulfat, oder die Verbindung der Formel I, wie in Anspruch 1 definiert, als Biozid.

**3.** Nicht-therapeutische Verwendung von 1,3-Dihydroxy-2-[hydroxyl(hydrogen-sulfooxy)methyl]propyl-dihydrogen-1,3-Disulfat, 1,3,4-Trihydroxy-2-[hydroxyl(hydrogen-sulfooxy)methyl]butyl-trihydrogen-1,3,4-Trisulfat, oder die Verbindung der Formel I, wie in Anspruch 1 definiert, als Chelatbildner.

**4.** Nicht-therapeutische Verwendung nach einem der Ansprüche 1-3, wobei die Verbindung nach Formel I ausgewählt ist aus:

- Tetrahydroxyethyl-dihydrogen-1,2-Disulfat
- 1,2,3,4-Tetrahydroxybutyl-tetrahydrogen-1,2,3,4-Tetrasulfat
- Ethyl-hexahydrogen-hexasulfat

**5.** Nicht-therapeutische Verwendung nach einem der Ansprüche 1-3, wobei M Natrium ist.

**6.** Nicht-therapeutische Verwendung nach einem der Ansprüche 1-5, wobei die Verbindung nach Formel I ausgewählt ist aus:

- Tetrahydroxyethyl-dinatrium-1,2-Disulfat
- 1,2,3,4-Tetrahydroxybutyl-tetranatrium-1,2,3,4-Tetrasulfat
- Ethyl-hexanatrium-hexasulfat (EHS-6Na).

**7.** 1,3-Dihydroxy-2-[hydroxyl(hydrogen-sulfooxy)methyl]propyl-dihydrogen-1,3-Disulfat, 1,3,4-Trihydroxy-2-[hydroxyl(hydrogen-sulfooxy)methyl]butyl-trihydrogen-1,3,4-Trisulfat, oder eine Verbindung der nachstehenden Formel I als Lockerungs- oder Öffnungsmittel für eine Zellwand-Membran von Mikroorganismen, um die Porosität der Membran zu vergrößern, zur Verwendung bei der Behandlung einer bakteriellen, viralen, Pilz- oder Algeninfektion,

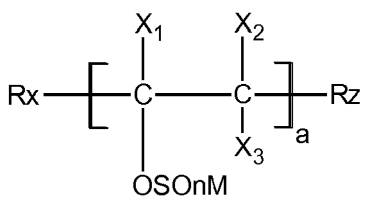

Formel I

wobei:

$X_1$, $X_2$, $X_3$ ausgewählt sind aus: Hydroxy, Halogenid, Sulfit, Sulfat, und Sulfonat, wobei $X_1$, $X_2$, $X_3$ gleiche oder verschiedene Atome oder Gruppen sein können;
Rx, Rz ausgewählt sind aus: Wasserstoff, Hydroxy, Alkyl $C_{1-20}$, Alkylen $C_{1-20}$, Alkylalkohol $C_{1-20}$ aliphatisch oder verzweigt substituiert oder unsubstituiert, Aryl, Cycloalkyl substituiert oder unsubstituiert, Sulfit, Sulfat und Sulfonat;

M: Wasserstoff, oder einGruppe I, Gruppe II, Übergangsgruppenkation ist, das pharamzeutisch verträglich ist.
n eine ganze Zahl von 0-3 ist
a eine ganze Zahl, ausgewählt aus 1 oder mehr
und/oder deren Salz, Solvat, Hydrat ist, das pharmazeutisch verträglich ist, und wobei die Verbindung der Formel I mindestens eine Sulfatgruppe umfasst.

**8.** 1,3-Dihydroxy-2-[hydroxyl(hydrogen-sulfooxy)methyl]propyl-dihydrogen-1,3-Disulfat, 1,3,4-Trihydroxy-2-[hydroxyl(hydrogen-sulfooxy)methyl]butyl-trihydrogen-1,3,4-Trisulfat, oder die Verbindung der Formel I, wie in Anspruch 7 definiert, zur Verwendung nach Anspruch 7 als Biozid.

**9.** Verbindung zur Verwendung nach einem der Ansprüche 7-8, wobei die
Verbindung nach Formel I ausgewählt ist aus:

- Tetrahydroxyethyl-dihydrogen-1,2-Disulfat
- 1,2,3,4-Tetrahydroxybutyl-tetrahydrogen-1,2,3,4-Tetrasulfat
- Ethyl-hexahydrogen-hexasulfat

**10.** Verbindung zur Verwendung nach einem der Ansprüche 7-8, wobei M Natrium ist.

**11.** Verbindung zur Verwendung nach einem der Ansprüche 7-10, wobei die Verbindung nach Formel I ausgewählt ist aus:

- Tetrahydroxyethyl-dinatrium-1,2-Disulfat
- 1,2,3,4-Tetrahydroxybutyl-tetranatrium-1,2,3,4-Tetrasulfat
- Ethyl-hexanatrium-hexasulfat (EHS-6Na).

**12.** Verbindung, die ausgewählt ist aus:

- Tetrahydroxyethyl-dihydrogen-1,2-Disulfat
- 1,2,3,4-Tetrahydroxybutyl-tetrahydrogen-1,2,3,4-Tetrasulfat
- 1,3-Dihydroxy-2-[hydroxyl(hydrogen-sulfooxy)methyl]propyl-dihydrogen-1,3-Disulfat
- 1,3,4-Trihydroxy-2-[hydroxyl(hydrogen-sulfooxy)methyl]butyl-trihydrogen-1,3,4-Trisulfat
- Ethyl-hexahydrogen-hexasulfat

**13.** Verbindung, die ausgewählt ist aus:

- Tetrahydroxyethyl-dinatrium-1,2-Disulfat
- 1,2,3,4-Tetrahydroxybutyl-tetranatrium-1,2,3,4-Tetrasulfat
- 1,3-Dihydroxy-2-[hydroxyl(natrium-sulfooxy)methyl]propyl-dinatrium-1,3-Disulfat
- 1,3,4-Trihydroxy-2-[hydroxyl(natrium-sulfooxy)methyl]butyl-trinatrium-1,3,4-Trisulfat.
- Ethyl-hexanatrium-hexasulfat (EHS-6Na).

**14.** Synthese der Verbindung nach Formel I, wie in Anspruch 1 definiert, oder nach einem der Ansprüche 12 oder 13, die den Schritt umfasst von:

- Umsetzen von Formaldehyd, Acetaldehyd, Ameisensäure, Essigsäure oder Oxalsäure mit Schwefelsäure ($H_2SO_4$) oder Sulfonsäure ($H_2SO_3$) und/oder Schwefeltrioxid (SO3) oder Schwefeldioxid ($SO_2$).

**15.** Nicht-therapeutisches Verfahren, um die Hydroxy-Hydroxy Bindung am Peptidoglycan oder Hydroxyl-Amid Bindung am Protein, umfasst in einer Zellwand-Membran eines Mikroorganismus oder einer anderen organischen Membran, zu brechen oder zu lösen, resultierend in einer Vergrößerung der Membranporosität durch Behandeln der Membran mit der Verbindung nach Formel I, wie in Anspruch 1 definiert, wobei die Verbindung die Membranporosität vergrößert, was die Membran zu vollständig durchlässig auf deren beiden Seiten umwandelt.

## Revendications

**1.** Utilisation non thérapeutique de 1,3-dihydroxy-2-[hydroxy(hydrogéno-sulfooxy)méthyl]propyl 1,3-disulfate de dihydrogène, de 1,3,4-trihydroxy-2-[hydroxy-(hydrogénosulfooxy)méthyl]butyl 1,3,4-trisulfate de trihydrogène, ou d'un composé de formule I ci-dessous, en tant qu'agent ouvrant ou relâchant la membrane de la paroi cellulaire de micro-organismes afin d'augmenter la porosité de la membrane

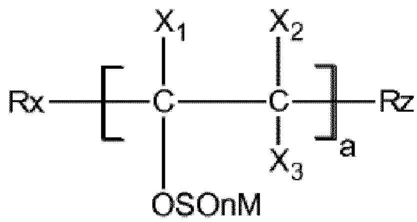

Formule I

formule dans laquelle :

$X_1$, $X_2$, $X_3$ sont choisis parmi : les groupes hydroxy, halogénure, sulfite, sulfate, et sulfonate, $X_1$, $X_2$, $X_3$ pouvant être des atomes ou groupes identiques ou différents ;
Rx, Rz sont choisis parmi : un atome d'hydrogène, des groupes hydroxy, alkyle en $C_1$-$C_{20}$, alkylène en $C_1$-$C_{20}$, alcool alkylique en $C_1$-$C_{20}$, aliphatique ou ramifié, substitué ou non substitué, aryle, cycloalkyle substitué ou non substitué, sulfite, sulfate, et sulfonate ;
M est un atome d'hydrogène, ou un cation du groupe I, du groupe II, des groupes de transition, qui est pharmaceutiquement acceptable ;
n est un nombre entier valant de 0 à 3
a est un nombre entier choisi parmi 1 ou plus
et/ou des sels, produits de solvatation, hydrates, pharmaceutiquement acceptables de tels composés, et dans laquelle ledit composé de formule I comprend au moins un groupe sulfate.

**2.** Utilisation non thérapeutique de 1,3-dihydroxy-2-[hydroxy(hydrogéno-sulfooxy)méthyl]propyl 1,3-disulfate de dihydrogène, de 1,3,4-trihydroxy-2-[hydroxy-(hydrogénosulfooxy)méthyl]butyl 1,3,4-trisulfate de trihydrogène, ou du composé de formule I telle que définie dans la revendication 1, en tant que biocide.

**3.** Utilisation non thérapeutique de 1,3-dihydroxy-2-[hydroxy(hydrogéno-sulfooxy)méthyl]propyl 1,3-disulfate de dihydrogène, de 1,3,4-trihydroxy-2-[hydroxy-(hydrogénosulfooxy)méthyl]butyl 1,3,4-trisulfate de trihydrogène, ou du composé de formule I telle que définie dans la revendication 1, en tant qu'agent chélateur.

**4.** Utilisation non thérapeutique selon l'une quelconque des revendications 1 à 3, dans laquelle le composé selon la formule I est choisi parmi :

- le tétrahydroxyéthyl 1,2-disulfate de dihydrogène
- le 1,2,3,4-tétrahydroxybutyl 1,2,3,4-tétrasulfate de tétrahydrogène
- l'éthyl hexasulfate d'hexahydrogène.

**5.** Utilisation non thérapeutique selon l'une quelconque des revendications 1 à 3, dans laquelle M est le sodium.

**6.** Utilisation non thérapeutique selon l'une quelconque des revendications 1 à 5, dans laquelle le composé selon la formule I est choisi parmi :

- le tétrahydroxyéthyl 1,2-disulfate de disodium
- le 1,2,3,4-tétrahydroxybutyl 1,2,3,4-tétrasulfate de tétrasodium
- l'éthyl hexasulfate d'hexasodium (EHS-6Na).

**7.** 1,3-dihydroxy-2-[hydroxy(hydrogénosulfooxy)méthyl]propyl 1,3-disulfate de dihydrogène, 1,3,4-trihydroxy-2-[hydroxy(hydrogénosulfooxy)méthyl]-butyl 1,3,4-trisulfate de trihydrogène, ou composé de formule I ci-dessous, en tant qu'agent ouvrant ou relâchant la membrane de la paroi cellulaire de micro-organismes afin d'augmenter la porosité de la membrane, destiné à l'utilisation dans le traitement d'une infection bactérienne, virale, fongique ou algale,

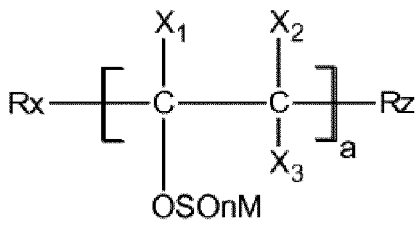

Formule I

formule dans laquelle :

$X_1$, $X_2$, $X_3$ sont choisis parmi : les groupes hydroxy, halogénure, sulfite, sulfate, et sulfonate, $X_1$, $X_2$, $X_3$ pouvant être des atomes ou groupes identiques ou différents ;
Rx, Rz sont choisis parmi : un atome d'hydrogène, des groupes hydroxy, alkyle en $C_1$-$C_{20}$, alkylène en $C_1$-$C_{20}$, alcool alkylique en $C_1$-$C_{20}$, aliphatique ou ramifié, substitué ou non substitué, aryle, cycloalkyle substitué ou non substitué, sulfite, sulfate, et sulfonate ;
M est un atome d'hydrogène, ou un cation du groupe I, du groupe II, des groupes de transition, qui est pharmaceutiquement acceptable ;
n est un nombre entier valant de 0 à 3
a est un nombre entier choisi parmi 1 ou plus
et/ou sels, produits de solvatation, hydrates, pharmaceutiquement acceptables de tels composés, et où ledit composé de formule I comprend au moins un groupe sulfate.

**8.** 1,3-dihydroxy-2-[hydroxy(hydrogénosulfooxy)méthyl]propyl 1,3-disulfate de dihydrogène, 1,3,4-trihydroxy-2-[hydroxy(hydrogénosulfooxy)méthyl]-butyl 1,3,4-trisulfate de trihydrogène, ou composé de formule I telle que définie dans la revendication 7, destiné à l'utilisation selon la revendication 7 en tant que biocide.

**9.** Composé destiné à l'utilisation selon l'une quelconque des revendications 7 et 8, où le composé selon la formule I est choisi parmi :

- le tétrahydroxyéthyl 1,2-disulfate de dihydrogène
- le 1,2,3,4-tétrahydroxybutyl 1,2,3,4-tétrasulfate de tétrahydrogène
- l'éthyl hexasulfate d'hexahydrogène.

**10.** Composé destiné à l'utilisation selon l'une quelconque des revendications 7 et 8, dans lequel M est le sodium.

**11.** Composé destiné à l'utilisation selon l'une quelconque des revendications 7 à 10, où le composé selon la formule I est choisi parmi :

- le tétrahydroxyéthyl 1,2-disulfate de disodium
- le 1,2,3,4-tétrahydroxybutyl 1,2,3,4-tétrasulfate de tétrasodium
- l'éthyl hexasulfate d'hexasodium (EHS-6Na).

**12.** Composé qui est choisi parmi :

- le tétrahydroxyéthyl 1,2-disulfate de dihydrogène
- le 1,2,3,4-tétrahydroxybutyl 1,2,3,4-tétrasulfate de tétrahydrogène
- le 1,3-dihydroxy-2-[hydroxy(hydrogénosulfooxy)méthyl]propyl 1,3-disulfate de dihydrogène,
- le 1,3,4-trihydroxy-2-[hydroxy(hydrogénosulfooxy)méthyl]butyl 1,3,4-trisulfate de trihydrogène
- l'éthyl hexasulfate d'hexahydrogène.

**13.** Composé qui est choisi parmi :

- le tétrahydroxyéthyl 1,2-disulfate de disodium
- le 1,2,3,4-tétrahydroxybutyl 1,2,3,4-tétrasulfate de tétrasodium
- le 1,3-dihydroxy-2-[hydroxy(sodiosulfooxy)méthyl]propyl 1,3-disulfate de disodium

- le 1,3,4-trihydroxy-2-[hydroxy(sodiosulfooxy)méthyl]butyl 1,3,4-trisulfate de trisodium
- l'éthyl hexasulfate d'hexasodium (EHS-6Na).

**14.** Synthèse du composé selon la formule I telle que définie dans la revendication 1 ou selon l'une quelconque des revendications 12 et 13, qui comprend l'étape consistant à :

- faire réagir du formaldéhyde, de l'acétaldéhyde, de l'acide formique, de l'acide acétique ou de l'acide oxalique avec de l'acide sulfurique ($H_2SO_4$) ou de l'acide sulfonique ($H_2SO_3$) et/ou du trioxyde de soufre ($SO_3$) ou du dioxyde de soufre ($SO_2$).

**15.** Procédé non thérapeutique pour rompre ou relâcher la liaison hydroxy-hydroxy sur un peptidoglycane ou la liaison hydroxy-amide sur une protéine compris(e) dans une membrane de la paroi cellulaire d'un micro-organisme ou une autre membrane organique, ce qui a pour résultat une augmentation de la porosité de la membrane, par traitement de ladite membrane par le composé selon la formule I telle que définie dans la revendication 1, dans lequel ledit composé augmente la porosité de la membrane, ce qui rend la membrane totalement perméable sur ses deux faces.

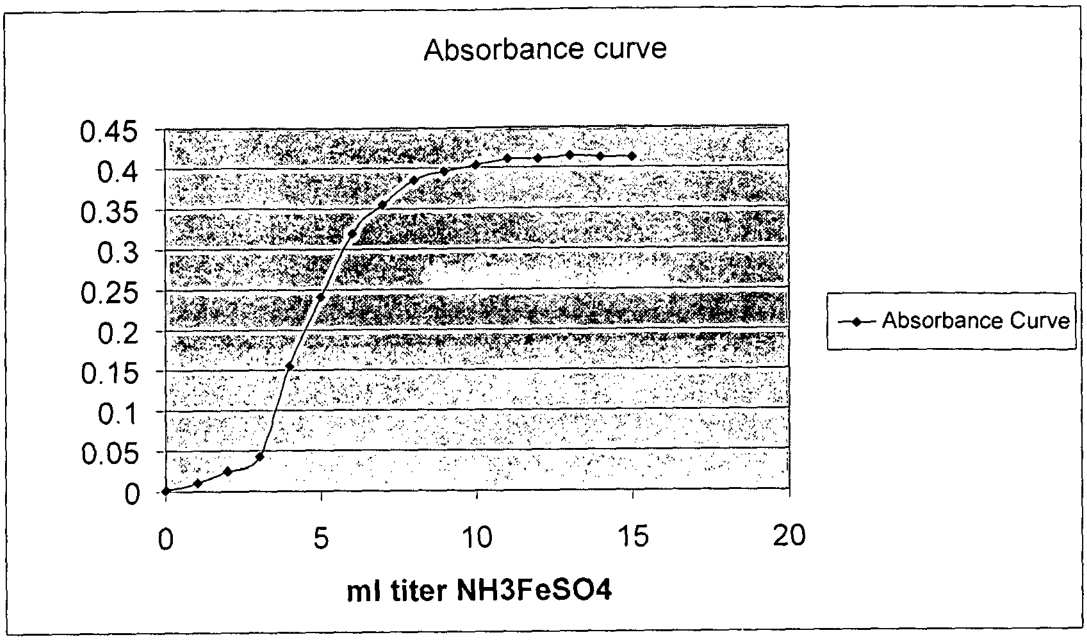
Absorbance curve
0.45
0.4
0.35
0.3
0.25
0.2
0.15
0.1
0.05
0
0
5
10
15
20
ml titer NH3FeSO4
Absorbance Curve

Figure 1

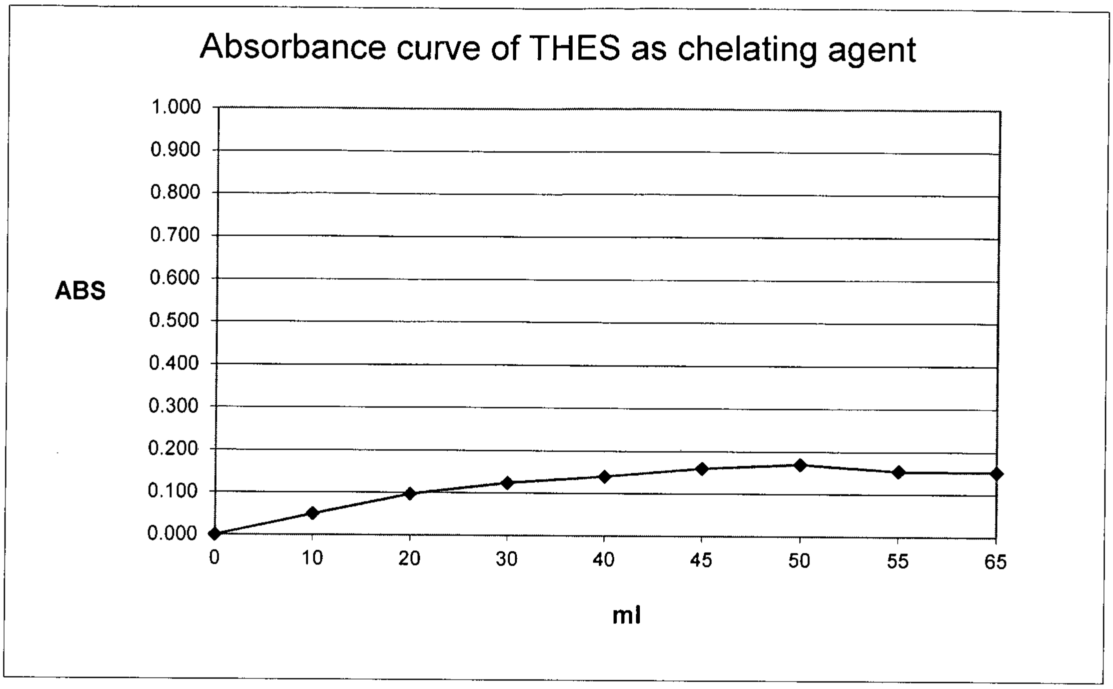
Absorbance curve of THES as chelating agent
ABS
1.000
0.900
0.800
0.700
0.600
0.500
0.400
0.300
0.200
0.100
0.000
0
10
20
30
40
45
50
55
65
ml

Figure 2a

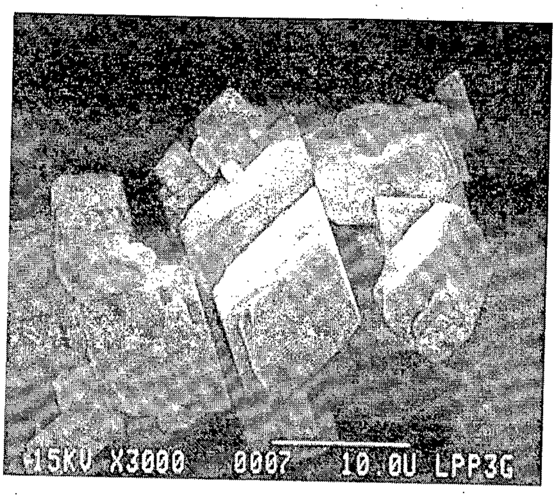


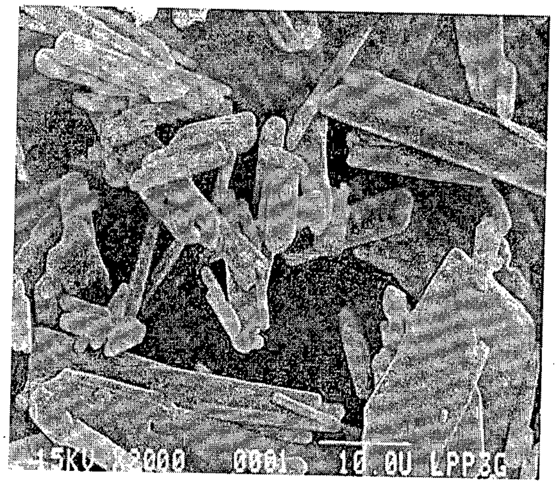


Figure 3

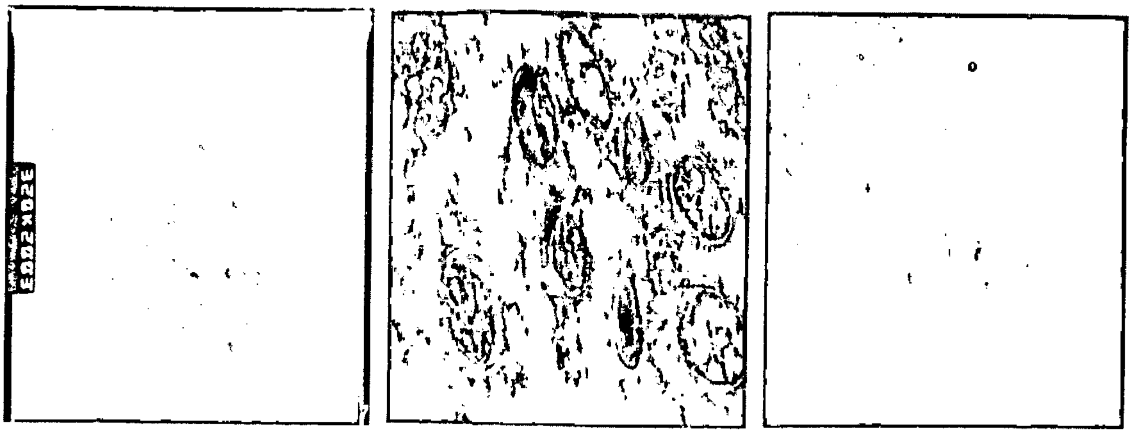

Figure 4

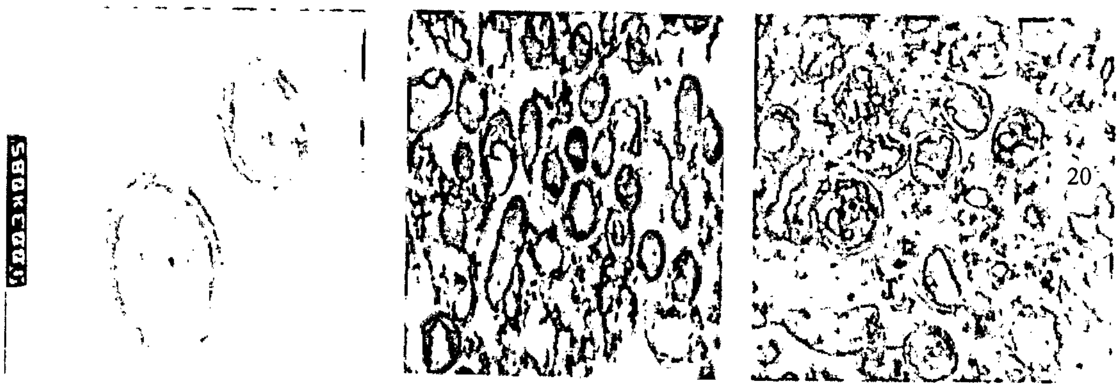


Figure 5

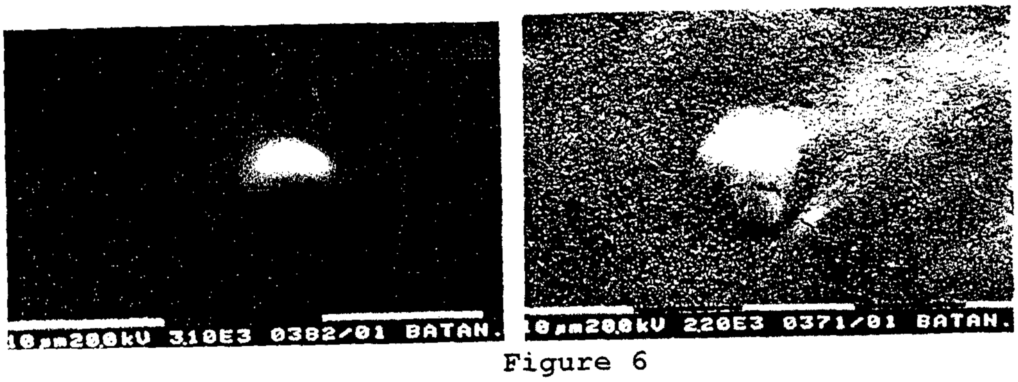


Figure 6

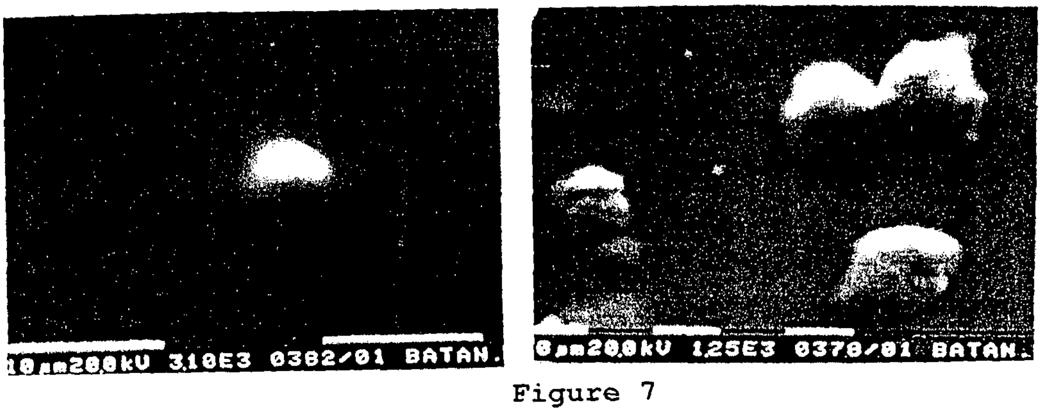


Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02081487 A1
- US 20030055007 A


### Non-patent literature cited in the description


- **H. HAQUE ; A. D. RUSSELL.** Effect of Chelating Agents on the Susceptibility of Some Strains of Gram-Negative Bacteria to Some Antibacterial Agents. *ANTIMICROBIAL AGENTS AND CHEMOTHERAPY,* August 1974, 200-206
- **KAUR P ; VADEHRA DV.** Effect of certain chelating agents on the antibacterial action of silver nitrate. *J Hyg Epidemiol Microbiol Immunol.,* 1988, vol. 32 (3), 299-306
- **A. HINTON JR. ; K.D. INGRAM.** Comparison of the Antibacterial Activity of Chelating Agents Using the Agar Diffusion Method. *International Journal of Poultry Science,* 2010, vol. 9 (11), 1023-1026